# EUROPÄISCHE PATENTSCHRIFT

(11) **EP 2 744 561 B1**
(45) Veröffentlichungstag und Bekanntmachung des Hinweises auf die Patenterteilung: **07.06.2023**
(21) Anmeldenummer: 12756358.3
(22) Anmeldetag: 14.08.2012
(51) Int. Cl.: A61N 1/06, A61N 1/05, A61N 1/32, A61B 18/14, A61B 18/00, A61N 1/36, A61M 25/00

(54) **VORRICHTUNG ZUR APPLIKATION EINER GEPULSTEN RADIOFREQUENZTHERAPIE IM GEFÄSSSYSTEM ODER ANDEREN KÖRPERHOHLRÄUMEN DES MENSCHLICHEN ODER TIERISCHEN KÖRPERS**
DEVICE FOR ADMINISTERING A PULSED RADIOFREQUENCY THERAPY IN THE VASCULAR SYSTEM OR IN OTHER BODY CAVITIES OF THE HUMAN OR ANIMAL BODY
DISPOSITIF D'APPLICATION D'UNE THÉRAPIE PAR RADIOFRÉQUENCE PULSÉE DANS LE SYSTÈME VASCULAIRE OU D'AUTRES CAVITÉS CORPORELLES DU CORPS HUMAIN OU ANIMAL

(30) Priorität: 19.08.2011 DE 102011110667
(43) Veröffentlichungstag der Anmeldung: 25.06.2014
(73) Patentinhaber: Omar, Omar-Pasha, 53797 Lohmar (DE)
(72) Erfinder: Omar, Omar-Pasha, 53797 Lohmar (DE)
(74) Vertreter: Hohendorf Kierdorf Patentanwälte PartGmbB
(86) Internationale Anmeldenummer: PCT/EP2012/003463
(87) Internationale Veröffentlichungsnummer: WO 2013/026540

(56) Entgegenhaltungen:
- DE-A1- 19 541 566
- DE-A1-102008 005 377
- DE-U1- 9 490 471
- DE-U1-202011 003 353
- US-A- 5 178 620
- US-A1- 2003 023 295
- US-A1- 2003 208 101
- US-A1- 2004 236 388
- US-A1- 2006 064 150
- US-A1- 2007 027 449
- US-A1- 2009 306 655
- US-A1- 2009 318 994

## Beschreibung

Die Erfindung betrifft eine Vorrichtung zur Applikation einer gepulsten Radiofrequenztherapie im Gefäßsystem oder anderen Körperhohlräumen des menschlichen oder tierischen Körpers, beispielsweise im Epiduralraum des Wirbelkanals, im Spinalraum des Wirbelkanals oder in Bereichen der Schädelhöhle.

Bei der Radiofrequenztherapie wird mittels einer Elektrode ein Wechselstrom in die Nähe eines Nervs oder im Gewebe abgegeben. Dieser Strom erzeugt im Gewebe bei kontinuierlicher Abgabe Hitze sowie ein elektrisches Feld. Durch die Hitzeeinwirkung wird ein anvisierter Nerv gereizt und/oder geschädigt, wodurch die durch den Nerv verursachten Schmerzen gelindert werden. Weiterhin wird das elektrische Feld distal ins zentrale Nervensystem weitergeleitet, wodurch die Neuroplastizität des zentralen Nervensystems beeinflusst wird. Dadurch wird eine weitere Schmerzlinderung erzielt. Eine Vorrichtung zur Applikation einer Radiofrequenztherapie umfasst einen Generator, eine Erdungsplatte mit Kabel sowie eine Elektrode, welche mittels einer speziellen, nicht isolierten Hohlkanüle eingeführt wird.

Im Gegensatz zu der konventionellen Radiofrequenztherapie wird eine gepulste Radiofrequenztherapie bei Körpertemperatur durchgeführt, so dass keine Hitzeschäden und Deafferenzierungsschmerzen verursacht werden. Die gepulste Radiofrequenztherapie erzeugt im Gewebe keine Hitze, da nur alle 0,5 sec Energie in kleinen Quanten von 20 msec zugeführt wird. Die Gewebetemperatur steigt dabei auf maximal 42 Grad. Durch die gepulste Radiofrequenztherapie werden in den Nervenzellen, unter anderem des Rückenmark-Hinterhorns, Gene aktiviert, die zu einer Dämpfung der schmerzafferenten Aktivität führen. Der 20 msec dauernde Strompuls hat dabei beispielsweise eine Frequenz von 500.000 Hz. Die angeführten Parameter, wie beispielsweise die Pulsbreite, können verändert werden, sofern eine schädigende Gewebetemperatur nicht erreicht wird.

Aus dem Stand der Technik sind Spezialnadeln bekannt, die an einen Generator für gepulste Hochfrequenz angeschlossen werden. Solche Spezialnadeln und Hochfrequenzgeneratoren werden verwendet, um durch gezielte Reizung von Nerven die Freisetzung von schmerzhemmenden Substanzen im Rückenmark auszulösen und dadurch eine Schmerzbehandlung zu bewirken. Der Einsatz dieser Spezialnadeln stößt jedoch häufig an anatomische Grenzen oder wird wegen der Gefahr der Verletzung beim Einführen der Spezialnadeln vermieden. Aus der EP 1 181 947 A2 ist ein Epiduralkatheter mit mindestens drei in Reihe angeordneten Elektroden bekannt. Die Elektroden dienen zur elektrischen Stimulation von Nerven oder des Rückenmarks. Es kann ein Kanal zur Verabreichung von Medikamenten vorgesehen sein, so dass zusätzlich zur elektrischen Stimulation des Rückenmarks oder der Spinalnerven eine Injektion schmerzstillender Medikamente erfolgen kann.

Die DE 203 12 110 U1 offenbart einen flexiblen Katheter, insbesondere Epiduralkatheter, der im proximalen Bereich mindestens zwei elektrische Kontakte aufweist, deren Zuleitung im Katheter angeordnet sind und im proximalen Bereich einen Temperatursensor aufweist, dessen Anschlussleitungen ebenfalls im Katheter angeordnet sind. Mittels des Temperatursensors kann eine durch die Stimulation verursachte oder durch die zugeführte elektrische Energie bewirkte Temperaturerhöhung überwacht werden.

Die vorbeschriebenen Vorrichtungen zur Applikation einer gepulsten Radiofrequenztherapie sind allesamt dazu ausgebildet, Nervengewebe zu behandeln. Dazu wird die Vorrichtung so in der Nähe des Nervs angeordnet, dass das elektrische Feld möglichst gezielt auf den Nerv einwirkt. Dazu sind die Elektroden ortsfest auf einem entsprechenden Katheter angeordnet. Die Eigenschaften des erzeugten elektrischen Feldes können somit nur mittels des Generators über die erzeugte Spannung angepasst werden, durch Änderung der Polarität erzeugten Spannung oder durch Einführen einer anderen Vorrichtung mit unterschiedlichen Elektronenabständen.

Aus dem Stand der Technik sind auch Vorrichtungen zur Ablation von Gewebe bekannt, mit einer auf einem Katheter angeordneten Elektrode und einer auf einer im Katheter verschiebbaren Sonde angeordneten Elektrode: z.B. US 2007/0027449 A1, US 2009/0306655 A1, DE 195 41 566 A1 und DE 94 90 471 U1. US 2009/0318994 zeigt ein Katheter mit verschiebbaren Elektroden zur Herzstimulation.

Der vorliegenden Erfindung liegt somit die Aufgabe zugrunde, eine Vorrichtung bereitzustellen, die zur Applikation einer gepulsten Radiofrequenztherapie im Gefäßsystem oder anderen Körperhohlräumen oder Gewebe des menschlichen oder tierischen Körpers geeignet ist, wobei die Eigenschaften des erzeugten elektromagnetischen Feldes möglichst einfach und variabel an unterschiedliche

Verwendungen innerhalb des Gefäßsystems oder anderen Körperhohlräumen des menschlichen oder tierischen Körpers angepasst werden können.

Die Aufgabe wird erfindungsgemäß gelöst durch eine Vorrichtung gemäß Anspruch 1. Bevorzugte Ausführungen werden durch die abhängigen Ansprüche definiert.

Ausführungen, Varianten und Beispiele in dieser Offenbarung, die nicht unter den Wortlaut der angehängten Ansprüche fallen, sind nicht Teil der Erfindung.

Zur Applikation der gepulsten Radiofrequenztherapie im Gefäßsystem oder anderen Körperhohlräumen des menschlichen oder tierischen Körpers wird der Katheter mit der wenigstens einen darauf angeordneten Elektrode in das Gefäßsystem oder einen anderen Körperhohlraum des menschlichen oder tierischen Körpers eingeführt. Nachfolgend wird die Sonde mit der wenigstens einen weiteren darauf angeordneten Elektrode durch den Katheter in das Gefäßsystem oder den anderen Körperhohlraum des menschlichen oder tierischen Körpers eingeführt. Nachfolgend wird mittels des Generators zwischen den Elektroden ein elektromagnetisches Feld erzeugt. Die Eigenschaften des elektromagnetischen Feldes können zum einen durch die vom Generator erzeugte gepulste Spannung und zum anderen durch die Einführtiefe der Sonde in den Katheter angepasst werden, wobei durch die Einführtiefe der Sonde der Abstand zwischen der wenigstens einen Elektrode des Katheters und der wenigstens einen weiteren Elektrode der Sonde einstellbar ist. Dadurch lassen sich die Eigenschaften des erzeugten elektromagnetischen Feldes auf einfache Art und Weise an die unterschiedlichen Verwendungen innerhalb des Gefäßsystems oder anderen Körperhohlräumen des menschlichen oder tierischen Körpers anpassen.

Nach einer Variante der Erfindung ist die Sonde flexibel oder starr. Ein Vorteil einer flexiblen Sonde ist, dass diese auch an schwer zugänglichen Bereichen des Gefäßsystems oder anderen Körperhohlräumen des menschlichen oder tierischen Körpers einsetzbar ist, wohingegen eine starre Sonde zielgenau an einer Stelle innerhalb des Gefäßsystems oder anderen Körperhohlräumen des menschlichen oder tierischen Körpers platziert werden kann.

Nach einer weiteren vorteilhaften Variante der Erfindung umfasst die Sonde einen Einspritzkanal, zur Einbringung von Substanzen in das Gefäßsystem oder anderen Körperhohlräumen des menschlichen oder tierischen Körpers. Somit können beispielsweise schmerzhemmende Substanzen innerhalb des Gefäßsystems oder anderen Körperhohlräumen des menschlichen oder tierischen Körpers freigesetzt werden.

In einer besonders vorteilhaften Variante der Erfindung umfasst die Sonde weiterhin einen oder mehrere Messvorrichtungen, beispielsweise zur Bestimmung einer Spannung, eines Widerstandes, einer Stromstärke und/oder einer Temperatur. Mittels der einen oder mehreren Messvorrichtungen können die für die gepulste Radiofrequenztherapie wichtigen Parameter während der Behandlung jederzeit überwacht und gegebenenfalls aufgezeichnet werden.

Die elektrischen Verbindungen, um die Elektroden mit dem Generator zu verbinden, sind zweckmäßiger Weise in den Katheter und/oder der Sonde angeordnet, wodurch diese beispielsweise vor äußeren Beschädigungen geschützt werden.

Nach einer besonders zweckmäßigen Variante der Erfindung sind die elektrischen Verbindungen in der Wandung des Katheters und/oder der Sonde integriert.

Nach einer weiteren Variante der Erfindung umfasst die Vorrichtung weiterhin eine mit dem Katheter verbindbare Einführhilfe, mittels welcher die Sonde in den Katheter eingeführt werden kann. Die Einführhilfe dient zum einen zum leichteren Einführen der Sonde in den Katheter und zum anderen kann die Verbindung zwischen Katheter und Sonde mittels der Einführhilfe besonders fluiddicht ausgebildet werden.

In einer besonders vorteilhaften Variante weist die Einführhilfe einen luftdichten zylindrischen oder schlauchförmigen Körper auf , wobei die Sonde innerhalb des zylindrischen oder schlauchförmigen Körpers angeordnet ist. Dadurch kann beispielsweise sichergestellt werden, dass die Sonde keimfrei bleibt und ohne Gefahr einer Kontamination variabel in das Gefäßsystem oder den anderen Körperhohlraum ein- oder ausgeführt werden kann. Die Sterilität der Vorrichtung, insbesondere der Sonde, ist dadurch jederzeit gewährleistet.

In einer Variante der Erfindung sind der Katheter und die Einführhilfe miteinander verschraubbar. Dazu weist beispielsweise der Katheter ein Innengewinde auf und die Einführhilfe ein Außengewinde. Dadurch wird eine fluiddichte Verbindung zwischen Katheter und Einführhilfe ausgebildet.

Gemäß einer besonders zweckmäßigen Variante ist die Verbindung zwischen Katheter und Einführhilfe zumindest teilweise elektrisch leitfähig. Somit können die elektrischen Leitungen zur Verbindung der Elektrode des Katheters mit dem Generator über die Einführhilfe beispielsweise zu einem Stecker geführt werden, welcher mit dem Generator über eine elektrische Leitung verbindbar ist.

Nach einer weiteren Variante umfasst die Sonde eine Anzeigeeinrichtung, zur Anzeige der Eindringtiefe der Sonde in den Katheter. Diese kann beispielsweise durch eine aufgedruckte Skala ausgebildet sein. Über die Eindringtiefe der Sonde in den Katheter kann der Abstand zwischen der Elektrode auf dem Katheter und der Elektrode auf der Sonde bestimmt werden und somit die Eigenschaften des erzeugten elektrischen Feldes zwischen den Elektroden.

Gemäß einer Variante der Erfindung ist das proximale Ende des Katheters flexibel ausgebildet, wodurch die Fluiddichtigkeit zwischen Katheter und Sonde beziehungsweise Katheter und Einführhilfe gewährleistet wird.

Nach einer Variante der Erfindung ist die wenigstens eine Elektrode des Katheters am distalen Ende des Katheters angeordnet. Dadurch wird die Eindringtiefe der Elektrode des Katheters in das Gefäßsystem oder den anderen Körperhohlraum des menschlichen oder tierischen Körpers maximiert.

Nach einer weiteren Variante der Erfindung umfasst die Sonde zwei Elektroden, die jeweils elektrisch mit dem Generator verbunden sind. Dadurch kann zwischen den zwei Elektroden der Sonde ein elektrisches Feld aufgebaut werden und jeweils zwischen einer der Elektroden der Sonde und der Elektrode auf dem Katheter ein gesondertes elektrisches Feld aufgebaut werden. Dadurch wird die Einsetzbarkeit und Flexibilität der erfindungsgemäßen Vorrichtung zur Applikation einer gepulsten Radiofrequenztherapie im Gefäßsystem oder anderen Körperhohlräumen des menschlichen oder tierischen Körpers weiter verbessert. In einer weiteren Variante umfasst die erfindungsgemäße Vorrichtung eine elektrisch mit dem Generator verbundene Hautelektrode zur Befestigung am menschlichen oder tierischen Körper. Zwischen der Hautelektrode und der Elektrode auf dem Katheter und/oder der Elektrode auf der Sonde kann ein zusätzliches elektrisches Feld erzeugt werden, zur Applikation einer gepulsten Radiofrequenztherapie im Gefäßsystem oder anderen Körperhohlräumen, des menschlichen oder tierischen Körpers.

Die Erfindung findet Anwendung in einem Verfahren zur Applikation einer gepulsten Radiofrequenztherapie im Gefäßsystem oder anderen Körperhohlräumen des menschlichen oder tierischen Körpers, umfassend die Schritte Einführen eines Katheters 2 in das Gefäßsystem oder den anderen Körperhohlraum des menschlichen oder tierischen Körpers; optionales Verbinden einer Einführhilfe 10 mit dem Katheter 2; Einführen der Sonde 4 in den Katheter 2 und somit in das Gefäßsystem oder den anderen Körperhohlraum des menschlichen oder tierischen Körpers; Anlegen eines elektrischen Feldes an die Elektroden 3, 5, 6 und Verfahren der Einführtiefe der Sonde 4.

Das Verfahren umfasst beispielsweise eine Erzeugung eines elektrischen Feldes zwischen den Elektroden für 20 ms und eine Pulspause von 480 ms, wobei die gesamte Behandlungsdauer bei ungefähr 240 s liegt. Die angelegte Wechselspannung kann zwischen 25 V und 100 V liegen und eine Leistung von 0,5 W bis 15 W aufweisen.

Das Verfahren beziehungsweise die erfindungsgemäße Vorrichtung kann beispielsweise zur Behandlung folgender Erkrankungen, Körperorgane und Systeme des menschlichen oder tierischen Körpers verwendet werden: Immunsystemerkrankungen, Erschöpfungszustände, Schlafstörungen, psychische Erkrankungen wie beispielsweise Depressionen, Infektionskrankheiten, Erkrankungen des Blutsystems, Behandlung von gutartigen und bösartigen Tumoren, Behandlung des zentralen und peripheren Nervensystems, Sudeksche Erkrankung, Fibromyalgie und weitere Erkrankungen des autonomen Nervensystems, Erkrankungen des Bewegungssystems, sowie zur Leistungssteigerung beispielsweise im Sport.

Nachfolgend wird die Erfindung anhand von in den Figuren dargestellten Ausführungsbeispielen näher erläutert. Es zeigen:
- Fig. 1: eine perspektivische Ansicht einer erfindungsgemäßen Vorrichtung,
- Fig. 2: eine Schnittansicht einer erfindungsgemäßen Vorrichtung mit Katheter, Einführhilfe und Sonde, und
- Fig. 3: eine Detailansicht der Einführhilfe und der Sonde aus Figur 2.

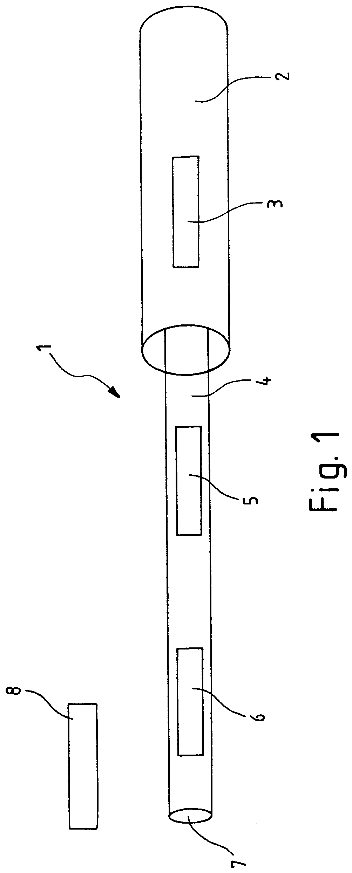

In Figur 1 ist eine Vorrichtung 1 zur Applikation einer gepulsten Radiofrequenztherapie im Gefäßsystem oder anderen Körperhohlräumen des menschlichen oder tierischen Körpers dargestellt. Die Vorrichtung 1 umfasst einen Katheter 2 und eine Sonde 4, wobei die Vorrichtung 1 mit einem Generator (nicht dargestellt) zur Erzeugung eines gepulsten elektromagnetischen Feldes zwischen elektrisch damit verbundenen Elektroden 3, 5, 6, 8 verbunden ist. Am distalen Ende des Katheters 2 ist eine erste Elektrode 3 angeordnet, welche elektrisch mit dem Generator verbindbar ist. Im distalen Bereich der Sonde 4 sind eine zweite Elektrode 5 und eine dritte Elektrode 6 angeordnet, welche ebenfalls elektrisch mit dem Generator verbindbar sind. Weiterhin kann die Vorrichtung 1 eine Hautelektrode 8 oder Nadelelektrode umfassen, welche ebenfalls elektrisch mit dem Generator verbindbar ist. Die Sonde 4 umfasst weiterhin einen Einspritzkanal 7, zur Einbringung einer Substanz, beispielsweise ein schmerzhemmendes Mittel, in das Gefäßsystem oder den anderen Körperhohlraum des menschlichen oder tierischen Körpers.

Mittels des Generators (nicht dargestellt) kann zwischen der ersten Elektrode 3, der zweiten Elektrode 5, der dritten Elektrode 6 und/oder der Hautelektrode 8 ein gepulstes elektromagnetisches Feld erzeugt werden, welches auf das Gefäßsystem oder den anderen Körperhohlraum des menschlichen oder tierischen Körpers einwirkt. Dazu wird der Katheter 2 in das Gefäßsystem oder den anderen Körperhohlraum des menschlichen oder tierischen Körpers eingebracht. Nachfolgend wird die Sonde 4 mittels des Katheters 2 in das Gefäßsystem oder den anderen Körperhohlraum des menschlichen oder tierischen Körpers eingeführt, wobei die Eigenschaften des elektrischen Feldes zwischen der ersten Elektrode 3, der zweiten Elektrode 5, der dritten Elektrode 6 und/oder der Hautelektrode 8 zum einen durch den Generator und zum anderen durch die Einführtiefe der Sonde 4 in den Katheter 2 eingestellt werden.

Das proximale Ende des Katheters 2, in welches die Sonde 4 eingeführt wird, ist flexibel ausgebildet, so dass die Verbindung zwischen Katheter 2 und Sonde 4 im distalen Bereich des Katheters 2 fluiddicht ist. Die Sonde 4 aus Figur 1 ist dabei starr ausgebildet, alternativ kann die Sonde 4 auch flexibel ausgebildet sein.

Die Sonde 4 umfasst weiterhin eine oder mehrere Messvorrichtungen (nicht dargestellt), beispielsweise zur Bestimmung einer Spannung, eines Widerstandes, einer Stromstärke und/oder einer Temperatur. Die Sonde 4 kann weiterhin eine Anzeigeeinrichtung (nicht dargestellt) umfassen, zur Anzeige der Eindringtiefe der Sonde 4 in den Katheter 2.

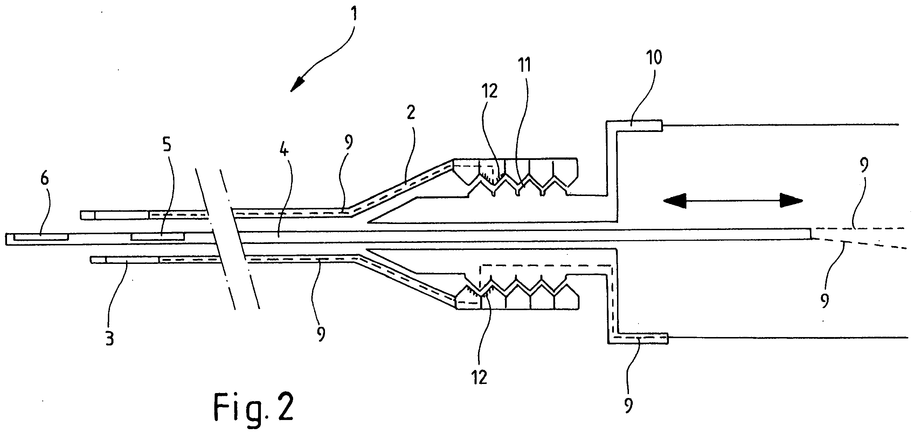

Figur 2 zeigt eine Schnittansicht einer erfindungsgemäßen Vorrichtung 1 mit Katheter 2, Einführhilfe 10 und Sonde 4. Mittels der Einführhilfe 10 kann die Sonde 4 in den Katheter 2 eingeführt werden und eine besonders fluiddichte Verbindung zwischen Katheter 2 und Einführhilfe 10 sowie zwischen Einführhilfe 10 und Sonde 4 ausgebildet werden. Der Katheter 2 und die Einführhilfe 10 weisen eine Verschraubung 11 auf zur Befestigung der Einführhilfe 10 an dem Katheter 2.

Die Einführhilfe 10 weist einen schlauchförmigen Körper auf, wobei die Sonde 4 innerhalb des schlauchförmigen Körpers angeordnet ist. Der schlauchförmige Körper ist luftdicht, so dass die Sonde 4 während einer Benutzung der erfindungsgemäßen Vorrichtung 1 nicht kontaminiert werden kann und keimfrei bleibt.

Die erste Elektrode 3 am distalen Ende des Katheters 2 ist über eine elektrische Verbindung 9 mit dem Generator verbindbar. Die elektrisch leitfähige Verbindung 9 ist dabei in der Wandung des Katheters 2 integriert und führt bis zu der Verschraubung 11 am proximalen Ende des Katheters 2. Im Bereich der Verschraubung 11 weist der Katheter 2 einen elektrischen Kontakt auf zur Herstellung einer elektrischen Verbindung 12 mit einer elektrisch leitfähigen Verbindung 9 in der Wandung der Einführhilfe 10.

Die zweite Elektrode 5 und die dritte Elektrode 6 im distalen Bereich der Sonde 4 sind mittels elektrisch leitfähiger Verbindungen 9 mit dem Generator verbunden, wobei die elektrisch leitfähigen Verbindungen 9 in der Sonde 4 angeordnet sind und am proximalen Ende der Sonde 4 aus der Sonde 4 austreten, um nachfolgend mit dem Generator verbunden zu werden.

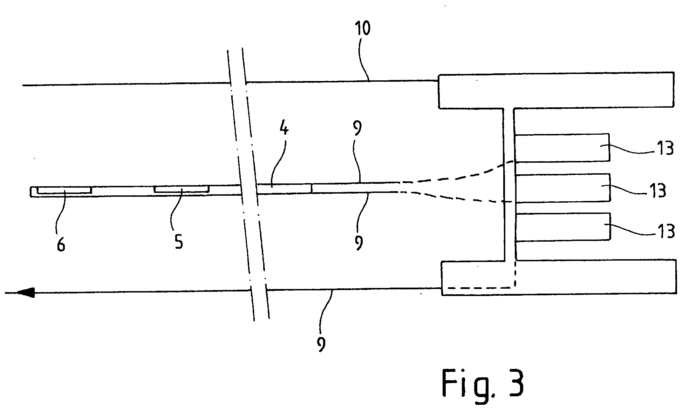

In Figur 3 ist eine Detailansicht der Einführhilfe 10 und der Sonde 4 aus Figur 2 dargestellt. Am distalen Ende der Einführhilfe 10 sind Steckverbinder 13 angeordnet, welche mit den elektrischen Verbindungen 9 zu den Elektroden 3, 5, 6 elektrisch leitfähig verbunden sind. Auf die Steckverbinder 13 kann ein entsprechend ausgebildeter Stecker aufgesteckt werden, zur Herstellung einer elektrisch leitfähigen Verbindung zwischen den Steckverbindern 13 und dem Generator, zur Erzeugung eines elektromagnetischen Feldes zwischen den Elektroden 3, 5,6.

Die Begriffe distal und proximal im Sinne der Erfindung sind aus Sicht eines Chirurgen zu verstehen, so dass das distale Ende das dem Patienten/Körper zugewandte Ende und das proximale Ende das dem Anwender/Chirurg zugewandte Ende der Vorrichtung ist.

### Bezugszeichenliste

- 1: Vorrichtung
- 2: Katheter
- 3: erste Elektrode
- 4: Sonde
- 5: zweite Elektrode
- 6: dritte Elektrode
- 7: Einspritzkanal
- 8: Hautelektrode
- 9: elektrische Verbindung
- 10: Einführhilfe
- 11: Verschraubung
- 12: elektrisch leitfähige Verbindung
- 13: Steckverbinder

## Patentansprüche

1. Vorrichtung (1) zur Applikation einer gepulsten Radiofrequenztherapie im Gefäßsystem oder anderen Körperhohlräumen des menschlichen oder tierischen Körpers umfassend:
einen Katheter (2) mit wenigstens einer darauf angeordneten Elektrode (3) zum Einbringen in das Gefäßsystem oder einen anderen Körperhohlraum des menschlichen oder tierischen Körpers;
eine Sonde (4) mit wenigstens einer weiteren darauf angeordneten Elektrode (5), (6), wobei die Sonde (4) durch den Katheter (2) in das Gefäßsystem oder den anderen Körperhohlraum des menschlichen oder tierischen Körpers einführbar ist; und
einen mit den Elektroden (3, 5, 6) elektrisch verbundenen Generator zur Erzeugung eines gepulsten elektromagnetischen Feldes zwischen den Elektroden (3, 5, 6), wobei der erzeugte Strompuls einen Wechselstrom mit einer Frequenz von 500.000 Hz aufweist, und wobei durch das erzeugte elektromagnetische Feld die Gewebetemperatur auf maximal 42°C steigt,
wobei die Eigenschaften des elektrischen Feldes zwischen den Elektroden (3, 5, 6) zum einen durch den Generator und zum anderen durch die Einführtiefe der Sonde (4) in den Katheter (2) einstellbar sind,
wobei durch die Einführtiefe der Sonde (4) der Abstand zwischen der wenigstens einen Elektrode (3) des Katheters (2) und der wenigstens einen weiteren Elektrode (5, 6) der Sonde (4) einstellbar ist.

2. Vorrichtung (1) nach Anspruch 1, wobei die Sonde (4) flexibel oder starr ausgebildet ist.

3. Vorrichtung (1) nach Anspruch 1 oder 2, wobei die Sonde (4) einen Einspritzkanal umfasst, zur Einbringung von Substanzen in das Gefäßsystem oder den anderen Körperhohlraum des menschlichen oder tierischen Körpers.

4. Vorrichtung (1) nach einem der Ansprüche 1 bis 3, wobei die Sonde (4) weiterhin eine oder mehrere Messvorrichtungen umfasst, beispielsweise zur Bestimmung einer Spannung, eines Widerstands, einer Stromstärke und/oder einer Temperatur.

5. Vorrichtung (1) nach einem der Ansprüche 1 bis 4, wobei in dem Katheter (2) und/oder der Sonde (4) elektrische Verbindungen (9) angeordnet sind, um die Elektroden (3, 5, 6) mit dem Generator zu verbinden.

6. Vorrichtung (1) nach Anspruch 5, wobei die elektrischen Verbindungen (9) in der Wandung des Katheters (2) und/oder der Sonde (4) integriert sind.

7. Vorrichtung (1) nach einem der Ansprüche 1 bis 6, wobei die Vorrichtung (1) weiterhin eine mit dem Katheter (2) verbindbare Einführhilfe (10) umfasst, mittels welcher die Sonde (4) in den Katheter (2) eingeführt werden kann, wobei die Einführhilfe (10) vorzugsweise einen zylindrischen oder schlauchförmigen Körper aufweist, innerhalb dessen die Sonde (4) angeordnet ist.

8. Vorrichtung (1) nach Anspruch 7, wobei der Katheter (2) und die Einführhilfe (10) miteinander verschraubbar sind.

9. Vorrichtung (1) nach Anspruch 7 oder 8, wobei die Verbindung (12) zwischen Katheter (2) und Einführhilfe (10) zumindest teilweise elektrisch leitfähig ist.

10. Vorrichtung nach einem der Ansprüche 1 bis 9, wobei die Sonde (4) eine Anzeigeeinrichtung umfasst, zur Anzeige der Eindringtiefe der Sonde (4) in den Katheter (2).

11. Vorrichtung (1) nach einem der Ansprüche 1 bis 10, wobei wenigstens das proximale Ende des Katheters (2) flexibel ausgebildet ist.

12. Vorrichtung (1) nach einem der Ansprüche 1 bis 11, wobei die wenigstens eine Elektrode (3) des Katheters (2) am distalen Ende des Katheters (2) angeordnet ist.

13. Vorrichtung (1) nach einem der Ansprüche 1 bis 12, wobei die Sonde (4) zwei Elektroden (5, 6) umfasst, die jeweils elektrisch mit dem Generator verbunden sind.

14. Vorrichtung (1) nach einem der Ansprüche 1 bis 13, weiterhin umfassend eine elektrisch mit dem Generator verbundene Hautelektrode (8), zur Befestigung am menschlichen oder tierischen Körper.

## Claims

1. Apparatus (1) for application of a pulsed radio frequency therapy in a vascular system or another cavity of the human or animal body comprising:
a catheter (2) with at least one electrode (3) disposed thereon for insertion into the vascular system or another cavity of the human or animal body;
a probe (4) with at least one further electrode (5, 6) disposed thereon, wherein the probe (4) is insertable via the catheter (2) into the vascular system or the other cavity of the human or animal body; and
a generator electrically connected with the electrodes (3, 5, 6) for generating a pulsed electro-magnetic field between the electrodes (3,5,6), wherein the generated current pulse comprises an alternating current having a frequency of 500.000 Hz and wherein the tissue temperature rises to a maximum of 42°C due to the generated electro-magnetic field,
wherein the characteristics of the electro-magnetic field between the electrodes (3, 5, 6) can be adapted on the one hand by the generator and on the other hand by the depth of penetration of the probe (4) into the catheter (2),
wherein the distance between the at least one electrode (3) of the catheter (2) and the at least one further electrode (5, 6) of the probe (4) is adaptable by the depth of penetration of the probe (4).

2. Apparatus (1) according to claim 1, wherein the probe (4) is flexible or rigid.

3. Apparatus (1) according to claim 1 or claim 2, wherein the probe (4) comprises an injection channel, for injecting a substance into the vascular system or the other cavity of the human or animal body.

4. Apparatus (1) according to one of claims 1 to 3, wherein the probe (4) further comprises one or more measuring apparatus, for example to determine a voltage, a resistance, a current and/or a temperature.

5. Apparatus (1) according to one of claims 1 to 4, wherein electrical connections (9) are disposed in the catheter (2) and/or probe (4), to connect the electrodes (3, 5, 6) with the generator.

6. Apparatus (1) according to claim 5, wherein the electrical connections (9) are integrated into the wall of the catheter (2) and/or the probe (4).

7. Apparatus (1) according to one of claims 1 to 6, wherein the apparatus (1) further comprises an insertion guide (10) connectable with the catheter (2), by which the probe (4) can be inserted into the catheter (2), wherein the insertion guide (10) preferably has a cylindrical or tube like body, in which the probe (4) is disposed.

8. Apparatus (1) according to claim 7, wherein the catheter (2) and the insertion guide (10) are screwable with one another.

9. Apparatus (1) according to claim 7 or 8, wherein the connection (12) between the catheter (2) and the insertion guide (10) is at least partially electrically conductive.

10. Apparatus (1) according to one of claims 1 to 9, wherein the probe (4) comprises an indicating device, for indicating the depth of penetration of the probe (4) in the catheter (2).

11. Apparatus (1) according to one of claims 1 to 10, wherein at least the proximal end of the catheter (2) is flexible.

12. Apparatus (1) according to one of claims 1 to 11, wherein the at least one electrode (3) of the catheter (2) is disposed at the distal end of the catheter (2).

13. Apparatus (1) according to one of claims 1 to 12, wherein the probe (4) comprises two electrodes (5, 6), which are each electrically connected with the generator.

14. Apparatus (1) according to one of claims 1 to 13, further comprising a skin electrode (8) electrically connected to the generator, for fixing at the human or animal body.

## Revendications

1. Appareil (1) pour l'application d'une thérapie par radiofréquence pulsée dans un système vasculaire ou une autre cavité du corps humain ou animal comprenant :
un cathéter (2) avec au moins une électrode (3) disposée dessus pour une insertion dans le système vasculaire ou une autre cavité du corps humain ou animal ;
une sonde (4) avec au moins une électrode supplémentaire (5, 6) disposée dessus, dans lequel la sonde (4) peut être insérée via le cathéter (2) dans le système vasculaire ou l'autre cavité du corps humain ou animal ; et
un générateur connecté électriquement avec les électrodes (3, 5, 6) pour générer un champ électromagnétique pulsé entre les électrodes (3, 5, 6), dans lequel l'impulsion de courant générée comprend un courant alternatif ayant une fréquence de 500 000 Hz et dans lequel la température de tissu s'élève à un maximum de 42 °C en raison du champ électromagnétique généré,
dans lequel les caractéristiques du champ électromagnétique entre les électrodes (3, 5, 6) peuvent être adaptées d'une part par le générateur et d'autre part par la profondeur de pénétration de la sonde (4) dans le cathéter (2),
dans lequel la distance entre l'au moins une électrode (3) du cathéter (2) et l'au moins une électrode supplémentaire (5, 6) de la sonde (4) peut être adaptée par la profondeur de pénétration de la sonde (4).

2. Appareil (1) selon la revendication 1, dans lequel la sonde (4) est flexible ou rigide.

3. Appareil (1) selon la revendication 1 ou la revendication 2, dans lequel la sonde (4) comprend un canal d'injection, permettant d'injecter une substance dans le système vasculaire ou l'autre cavité du corps humain ou animal.

4. Appareil (1) selon l'une des revendications 1 à 3, dans lequel la sonde (4) comprend en outre un ou plusieurs appareils de mesure, par exemple pour déterminer une tension, une résistance, un courant et/ou une température.

5. Appareil (1) selon l'une des revendications 1 à 4, dans lequel des connexions électriques (9) sont disposées dans le cathéter (2) etlou la sonde (4), pour connecter les électrodes (3, 5, 6) avec le générateur.

6. Appareil (1) selon la revendication 5, dans lequel les connexions électriques (9) sont intégrées dans la paroi du cathéter (2) et/ou de la sonde (4).

7. Appareil (1) selon l'une des revendications 1 à 6, dans lequel l'appareil (1) comprend en outre un guide d'insertion (10) pouvant être connecté avec le cathéter (2), grâce auquel la sonde (4) peut être insérée dans le cathéter (2), dans lequel le guide d'insertion (10) comporte de préférence un corps de type tube ou cylindrique, dans lequel la sonde (4) est disposée.

8. Appareil (1) selon la revendication 7, dans lequel le cathéter (2) et le guide d'insertion (10) peuvent être vissés l'un avec l'autre.

9. Appareil (1) selon la revendication 7 ou 8, dans lequel la connexion (12) entre le cathéter (2) et le guide d'insertion (10) est au moins partiellement électriquement conductrice.

10. Appareil (1) selon l'une des revendications 1 à 9, dans lequel la sonde (4) comprend un dispositif indicateur, permettant d'indiquer la profondeur de pénétration de la sonde (4) dans le cathéter (2).

11. Appareil (1) selon l'une des revendications 1 à 10, dans lequel au moins l'extrémité proximale du cathéter (2) est flexible.

12. Appareil (1) selon l'une des revendications 1 à 11, dans lequel l'au moins une électrode (3) du cathéter (2) est disposée au niveau de l'extrémité distale du cathéter (2).

13. Appareil (1) selon l'une des revendications 1 à 12, dans lequel la sonde (4) comprend deux électrodes (5, 6), qui sont chacune connectées électriquement avec le générateur.

14. Appareil (1) selon l'une des revendications 1 à 13, comprenant en outre une électrode cutanée (8) connectée électriquement au générateur, permettant de se fixer au corps humain ou animal.
